# EUROPÄISCHE PATENTSCHRIFT

(11) **EP 2 070 495 B1**
(45) Veröffentlichungstag und Bekanntmachung des Hinweises auf die Patenterteilung: **05.01.2011**
(21) Anmeldenummer: 08019728.8
(22) Anmeldetag: 12.11.2008
(51) Int. Cl.: A61F 5/02

(54) **Orthopädische Stützeinrichtung für einen menschlichen Rücken- und Lumbalbereich**
Orthopaedic support device for human back and lumbar regions
Dispositif de soutien orthopédique pour une zone dorsale et lombaire humaine

(30) Priorität: 14.12.2007 DE 102007062274
(43) Veröffentlichungstag der Anmeldung: 17.06.2009
(73) Patentinhaber: Bort GmbH, 71384 Weinstadt (DE)
(72) Erfinder: Strnad, Dieter, 68549 Ilvesheim (DE)
(74) Vertreter: Wilhelm, Peter

(56) Entgegenhaltungen:
- DE-A1- 10 329 454
- US-A- 903 403
- US-A- 5 868 694
- US-B1- 6 280 405

## Beschreibung

Die Erfindung betrifft eine orthopädische Stützeinrichtung für einen menschlichen Rücken- und Lumbalbereich mit einer Lumbalbandage sowie mit einer Trägergurtanordnung, die wenigstens einen dorsalen Gurtabschnitt sowie zwei über Schulterbereiche geführte Gurtabschnitte umfasst, und die mit der Lumbalbandage verbunden ist, wobei wenigstens einem Gurtabschnitt ein elastisch nachgiebiger Kraftbegrenzungsteil zugeordnet ist.

Eine derartige Stützeinrichtung dient insbesondere als Osteoporose-Orthese.

Eine Osteoporose-Orthese ist aus der EP 0 941 721 A1 bekannt. Dort ist eine Leibbandage in Form einer Lumbalbandage vorgesehen, die mit einer über Schulterbereiche des Patienten verlaufenden Trägergurtanordnung verbunden ist. Die Trägergurtanordnung weist ein längs einer Wirbelsäule zum Nacken nach oben verlaufendes Rückenband auf, an das in dem Nackenbereich zwei Schultergurtabschnitte anschließen, die nach vorne über die Schulterbereiche und unter den Achseln hindurch wieder nach hinten bis auf Höhe des Lendenwirbelbereiches geführt sind. In diesem Bereich sind die Schultergurtabschnitte dorsal mit der Lumbalbandage verbunden.

Aus der DE 103 29 454 A1 ist eine weitere orthopädische Stützeinrichtung für den Rücken- und Lumbalbereich eines Patienten vorgesehen, die ebenfalls eine Lumbalbandage umfasst. Dorsal in die Lumbalbandage integriert ist eine Rückenorthese, die eine längs einer Wirbelsäule nach oben erstreckte Rückenpelotte umfasst. Die Stützeinrichtung weist zudem eine Trägergurtanordnung auf, die zwei Schultergurtabschnitte und zwei Leibgurtabschnitte umfasst. Sowohl die Schultergurtabschnitte als auch die Leibgurtabschnitte greifen dorsal an der Rückenpelotte der Rückenorthese an. Die Schultergurtabschnitte gehen - ebenfalls dorsal - in die Leibgurtabschnitte über, die wiederum frontseitig an der Lumbalbandage befestigt sind.

Die US 5,686,694 A, die als nächste Stand der Technik angesehen wird, offenbart eine Rückenstützeinrichtung mit einer Brustbandage, an der über die Schultern eines Patienten verlaufende Trägergurte angeordnet sind. An den Schulterträgergurten greifen elastisch nachgiebige Zugmittel an, die in einem formstabilen Stützrahmen umgelenkt sind. Sobald die Brustbandage angelegt ist und auf die Schultergurte durch die Zugmittel eine Zugkraft nach oben ausgeübt wird, wird für eine Sitzposition des Patienten eine Entlastung des unteren Rückenbereiches erzielt.

Aufgabe der Erfindung ist es, eine orthopädische Stützeinrichtung der Eingangs genannten Art zu schaffen, die begrenzt dynamisch Oberkörperbewegungen des Patienten folgen kann.

Diese Aufgabe wird durch die Merkmale des Patentanspruches 1 gelöst. Vorzugsweise ist jedem Gurtabschnitt ein elastisch nachgiebiger Kraftbegrenzungsteil zugeordnet. Vorzugsweise ist der zugehörige Gurtabschnitt selbst unelastisch ausgeführt, so dass Zugspannungen des Gurtabschnittes zuverlässig über die Spannung des Kraftbegrenzungsteiles einstellbar sind. Falls der Gurtabschnitt selbst elastisch ausgeführt ist, muss seine elastische Spannungsfähigkeit erfasst und auf den Kraftbegrenzungsteil abgestimmt werden. Der elastisch nachgiebige Kraftbegrenzungsteil ermöglicht für den jeweiligen Gurtabschnitt eine kraftbegrenzte elastische Nachgiebigkeit, so dass der Patient bestimmte Oberkörperbewegungen wie Rumpfbeugungen nach vorne oder Rumpfdrehungen nach links oder rechts in eingeschränktem Maße durchführen kann, wobei - abhängig von der jeweiligen Bewegung - die entsprechenden Gurtabschnitte gedehnt werden. Die Kraftbegrenzungsteile gewährleisten zum Einen, dass die elastische Nachgiebigkeit lediglich über einen einstellbaren Dehnungsweg erfolgt. Zum Anderen gewährleisten die Kraftbegrenzungsteile, dass jeder Bewegung durch die elastisch nachgiebigen Kraftbegrenzungsteile eine definierte Gegenkraft entgegengesetzt wird. Hierdurch wird eine gewünschte Muskelbeanspruchung des Patienten erreicht.

Die erfindungsgemäße Lösung kann schützend, stützend, überbrückend und/oder therapierend eingesetzt werden.

Vorzugsweise greifen alle Gurtabschnitte dorsal im Bereich der Lumbalbandage an. Dabei können die Gurtabschnitte entweder direkt mit der Lumbalbandage oder indirekt mit dieser verbunden sein, indem sie zumindest teilweise an einem Rückenorthesenteil angreifen, das wiederum mit der Lumbalbandage verbunden ist. Vorzugsweise sind alle Kraftbegrenzungsteile, insbesondere in Form von Zugfederelementen, auf Höhe der Lumbalbandage dorsal angeordnet und an der Lumbalbandage oder an einem mit der Lumbalbandage verbundenen Rückenstützteil gehalten.

In Ausgestaltung der Erfindung umfasst der Kraftbegrenzungsteil ein Zug- oder Druckfederelement. Vorzugsweise ist eine Schraubenzugfeder vorgesehen. Wesentlich ist es, dass der jeweilige Gurtabschnitt bei einer Bedienung gegenkraft- und insbesondere federkraftbeaufschlagt ist. Demzufolge werden auf die Gurtabschnitte regelmäßig lediglich Zugkräfte wirken. Denn der jeweilige Kraftbegrenzungsteil soll einer Längung des Gurtabschnittes durch eine definierte Gegenkraft entgegenwirken, so dass eine Längung lediglich gegen die definierte Zugkraft ermöglicht ist. Falls die Gurtabschnitte so ausgelegt sind, dass sie auch Druckbelastungen übertragen können, ist es grundsätzlich auch möglich, als Kraftbegrenzungsteil ein Druckfederelement vorzusehen.

In weiterer Ausgestaltung der Erfindung ist dorsal mit der Lumbalbandage eine zumindest weitgehend formsteife Stützplatte verbunden, an der die dorsalen Endbereiche der Gurtabschnitte angreifen. Die Stützplatte erstreckt sich vorzugsweise von einem Lendenbereich längs eines Wirbelsäulenbereichs nach oben. Vorzugsweise weist sie zumindest für einen unteren und oberen Bereich der Wirbelsäule die Funktion einer Rückenorthese auf.

In weiterer Ausgestaltung der Erfindung ist der wenigstens eine Kraftbegrenzungsteil austauschbar zwischen Stützplatte und zugehörigem Gurtabschnitt angeordnet. Dadurch ist es möglich, je nach gewünschter Therapierung, unterschiedliche Kraftbegrenzungsteile mit höheren oder geringeren Zugkräften und mit größeren oder geringeren Dehnungswegen einzusetzen.

In weiterer Ausgestaltung der Erfindung ist wenigstens der Gurtabschnitt, dem der Kraftbegrenzungsteil zugeordnet ist, unelastisch ausgeführt. Diese Ausgestaltung ist vorteilhaft, um eine klare Definition der Dehnungskräfte der Gurtabschnitte über die Kraftbegrenzungsteile zu ermöglichen.

In weiterer Ausgestaltung der Erfindung sind Mittel zur Einstellung einer Zugspannung des wenigstens einen Kraftbegrenzungsteiles und des wenigstens einen zugeordneten Gurtabschnittes vorgesehen. In vorteilhafter Weise ist es möglich, über diese Einstellmittel die Federkraft des Kraftbegrenzungsteils, d.h. die Dehnungskraft der Gurtabschnitte und die maximal zulässige Längung jedes Gurtabschnittes einzustellen. Hierzu umfassen die Mittel zur Einstellung der Gurtspannung in vorteilhafter Weise Mittel zur Längeneinstellung der Gurtabschnitte.

In weiterer Ausgestaltung der Erfindung umfassen die Mittel zur Einstellung der Gurtspannung wenigstens einen Schnellverschluss. Hierdurch ist eine rasche Fixierung der eingestellten Gurtspannung erzielbar.

In weiterer Ausgestaltung der Erfindung ist dem Schnellverschluss ein flexibler Verbindungsstreifen zugeordnet, dessen Länge größer ist als eine Gesamtlänge des Schnellverschlusses, und der im Bereich des Schnellverschlusses benachbarte Endbereiche jeweils eines Gurtabschnittes miteinander derart verbindet, dass die Länge des Gurtabschnittes bei geöffnetem Schnellverschluss größer ist als bei geschlossenem Schnellverschluss. Diese Ausgestaltung gilt insbesondere als Anlegehilfe für einen Patienten. Denn hierdurch kann ein Patient die orthopädische Stützeinrichtung zunächst zumindest weitgehend spannungsfrei selbst anziehen, bevor dann eine Hilfsperson die Schnellverschlüsse schließt und hierdurch die für die Therapierung gewünschte Gurtspannung aufbauen kann. Diese Ausgestaltung ist insbesondere aus dem Grund vorteilhaft, da häufig Patienten selbst nicht in der Lage sind, die vergleichsweise hohen Kräfte aufzubringen, um die orthopädische Stützeinrichtung in die gespannte Funktionsstellung zu bringen. Die Ausgestaltung ermöglicht ein einfaches Anziehen für den Patienten und ein anschließendes Spannen durch eine Hilfsperson.

In weiterer Ausgestaltung der Erfindung ist als Schnellverschluss ein mechanischer Steck-/Rastverschluss vorgesehen, der in Längsrichtung des Gurtabschnittes werkzeuglos zu schließen oder zu öffnen ist. In einfacher Weise ist in benachbarten Endbereichen eines Gurtabschnittes ein Steck- und ein Aufnahmeteil des Schnellverschlusses zugeordnet, die durch eine einfache Steckbewegung in Längsrichtung des jeweiligen Gurtabschnittes miteinander verbunden werden können. Die werkzeuglose Verbindung ermöglicht ein einfaches Zusammenstecken oder ein einfaches Lösen.

Weitere Vorteile und Merkmale der Erfindung ergeben sich aus den Ansprüchen.Nachfolgend ist ein bevorzugtes Ausführungsbeispiel der Erfindung beschrieben und anhand der Zeichnungen dargestellt.
Fig. 1 zeigt eine Rückansicht einer Ausführungsform einer erfindungsgemäßen Stützeinrichtung,
Fig. 2 eine Frontansicht der Stützeinrichtung nach Fig. 1, und
Fig. 3 in vergrößerter Darstellung den Ausschnitt III der Stützeinrichtung nach Fig. 2.

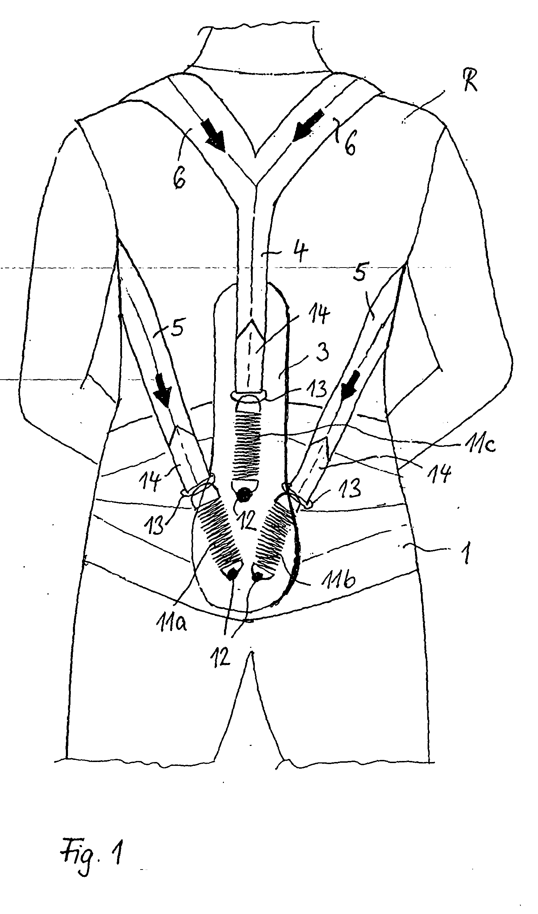

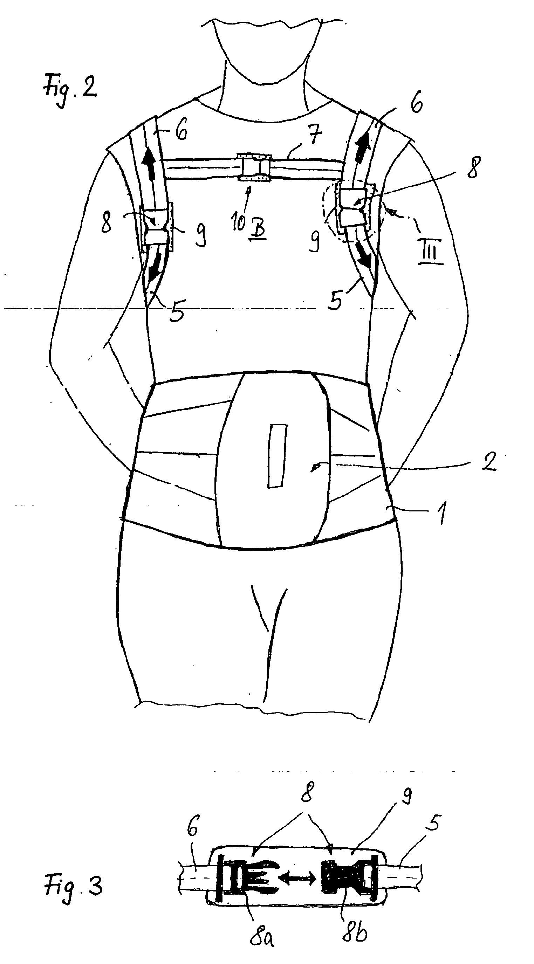

Eine orthopädische Stützeinrichtung in Form einer Osteoporose-Orthese nach den Figuren 1 bis 3 weist eine Lumbalbandage 1 auf, die als Leibbandage um einen Hüft- und Lendenbereich eines Patienten herumgelegt ist. Über einen Verschlussbereich 2 kann die Lumbalbandage 1 vom Körper entfernt werden. In Fig. 1 ist eine dorsale Ansicht der Stützeinrichtung gezeigt. In Fig. 2 eine frontale Ansicht.

Mit der Leibbandage 1 dorsal fest verbunden ist eine als Rückenpelotte gestaltete Rückenstützplatte 3, die sich zentral längs einer Wirbelsäule erstreckt. Die Rückenpelotte 3 ist durch Vernähen, Verkleben oder andere Verbindungsmittel mit dem Dorsalbereich der Lumbalbandage 1 verbunden. Die Rückenpelotte 3 ist vorzugsweise aus einem zumindest weitgehend formsteifen Kunststoffmaterial hergestellt und kann - wie auch die Lumbalbandage 1 - mit einer geeigneten Polsterung versehen sein.

Bei einer nicht dargestellten Ausführungsform weist die Rückenstützplatte keine eigenständige orthopädische Stützfunktion auf. Vielmehr dient sie lediglich als Träger- oder Lagerplatte für die nachfolgend näher beschriebene Trägergurtanordnung 4, 5, 6.

An einem unteren Bereich der Stützplatte 3 greifen zwei dorsale Schultergurtabschnitte 5 an, die sich von einem Rückenbereich R des Patienten aus unter dessen Achseln hindurch nach Art von Rucksackträgern nach vorne erstrecken und in einem Brustbereich B des Patienten in frontseitige Schultergurtabschnitte 6 übergehen. Die frontseitigen Schultergurtabschnitte 6 erstrecken sich über die Schultern des Patienten hinweg zum Rückenbereich R und münden dort gemeinsam in einen Rückengurtabschnitt 4, dessen unterer Endbereich ebenfalls mit der Stützplatte 3 verbunden ist.

Alle Gurtabschnitte 4, 5, 6 der Trägergurtanordnung sind über jeweils ein Zugfederelement 11a bis 11c an jeweils einem Lagerpunkt der Stützplatte 3 befestigt. Die Zugfederelemente 11a bis 11c dienen als Kraftbegrenzungsteile im Sinne der Erfindung. Die Lagerpunkte 12 stellen Gelenkpunkte für die Anlenkung der Zugfederelemente 11a bis 11c und damit für die Anbindung der Gurtabschnitte 4 bis 6 dar, so dass die Lagerpunkte 12 nach Art von Drehgelenken Ausgleichsbewegungen der angelenkten Zugfederelemente 11a bis 11 c und der zugeordneten Gurtabschnitte 4 bis 6 ermöglichen. Dadurch richten die Zugfederelemente 11a bis 11c sich immer längs zur jeweils wirkenden Kraftrichtung aus.

Eine Länge jedes Gurtabschnittes 4, 5 ist einstellbar. Hierzu weisen die dorsalen Gurtabschnitte 4, 5 Gurtenden 14 auf, die um entsprechende Umlenkbügel 13 umgelenkt und insbesondere durch Haftverschlüsse, wie Klettverschlüsse, fixiert sind. Die Zugfederelemente 11a bis 11c sind austauschbar angeordnet, so das sie durch stärkere oder schwächere Zugfederelemente ersetzt werden können. Beim dargestellten Ausführungsbeispiel wird jedes Zugfederelement 11a bis 11c durch eine Schraubenzugfeder gebildet.

Die über die Schultern verlaufenden Gurtabschnitte 6 sind im Brustbereich B des Patienten über einen Quergurt 7 miteinander verbunden. Sowohl der Quergurt 7 als auch alle Gurtabschnitte 4 bis 6 sind unelastisch ausgeführt, so dass eine elastische Nachgiebigkeit der Gurtabschnitte 4 bis 6 lediglich über eine entsprechende Längung der Zugfederelemente 11a bis 11c ermöglicht ist. Diese erzielen gleichzeitig zwangsläufig bei einer Längung eine Gegenkraft auf die entsprechenden Gurtabschnitte 4 bis 6. Die beiden Schultergurtabschnitte 5, 6 sind auf der Frontseite und damit auf Höhe des Brustbereiches B des Patienten über einen Schnellverschluss 8 miteinander verbunden, der anhand der Fig. 3 näher dargestellt ist. Jeder der beiden Schnellverschlüsse 8 weist zwei Verschlussteile 8a, 8b auf, die durch eine Steck-/Rastverbindung miteinander in Eingriff gebracht werden können. Derartige Steck/Rastverschlüsse sind grundsätzlich bekannt. Der als Stecker dienende Verschlussteil 8a wird in den als Aufnahmeteil dienenden Verschlussteil 8b in Längsrichtung der Gurtabschnitte 5, 6 eingesteckt. Beide Verschlussteile 8a, 8b sind jeweils an einem Endbereich des jeweiligen Gurtabschnittes 5, 6 angeordnet. Die beiden Endbereiche der benachbarten Gurtabschnitte 5, 6 sind jeweils über einen Verbindungsstreifen 9 miteinander verbunden, dessen Länge - in Längsrichtung der Gurtabschnitte 5, 6 gesehen - wesentlich länger ist als die Längserstreckung des Schnellverschlusses 8 in seiner zusammengefügten Rastposition. Der Verbindungsstreifen 9 ist aus flexiblem Material, insbesondere aus Textilmaterial, und dient dazu, in geöffnetem Zustand der Verschlussteile-8a, 8b dennoch die beiden Endbereiche der Gurtabschnitte 5, 6 miteinander zu verbinden. Die Endbereiche der benachbarten Gurtabschnitte 5, 6 sind somit in jedem Fall über den jeweiligen Verbindungsstreifen 9 miteinander verbunden. Bei geschlossenem Schnellverschluss 8 ist der Verbindungsstreifen 9 schlaff und in Bezug auf eine Zugkraftwirkung außer Funktion. Zugkräfte werden in der geschlossenen Stellung des Schnellverschlusses ausschließlich über den Schnellverschluss 8 zwischen den Gurtabschnitten 5, 6 übertragen. Jeder Verbindungsstreifen 9 dient dazu, auch bei geöffnetem Schnellverschluss 8 geschlossene Schultergurtabschnitte 5, 6 beizubehalten, so dass eine entsprechende Person die Schultergurtabschnitte 5, 6 nach Art von Rucksackträgern anziehen kann, ohne dass die Gurtabschnitte 5, 6 bereits unter Spannung gesetzt sind. Die Verbindungsstreifen 9 dienen somit als Anziehhilfe für die Osteoporose-Orthese.

Die Voreinstellung der Längen der Gurtabschnitte 4 bis 6 über die bereits beschriebenen Mittel zur Längeneinstellung 13, 14 wird derart auf die Schulter- und Rückenabmessung des jeweiligen Patienten abgestimmt, dass mit dem Schließen der Schnellverschlüsse 8 die gewünschte Spannung der Gurtabschnitte 4 bis 6 erzielt wird. Die Osteoporose-Orthese kann somit individuell auf die jeweilige individuelle Therapie des jeweiligen Patienten abgestimmt werden.

Auch der Quergurt 7 ist mit einem Schnellverschluss 10 versehen, dem ein analoger Verbindungsstreifen zugeordnet ist. In gespanntem Zustand, d.h. bei geschlossenem Schnellverschluss 10 dient der Quergurt 7 dazu, den gewünschten Gurtverlauf der Schultergurtabschnitte 6 über die Schultern des Patienten zu sichern und so zu vermeiden, dass die Schultergurtabschnitte 6 über die Schultern des Patienten nach außen abgleiten. Auch der Quergurt 7 ist mit Längeneinstellmitteln versehen, die vorliegend nicht dargestellt sind.

Die Länge der Gurtabschnitte 4 bis 6 wird derart auf die individuellen Körpermaße des jeweiligen Patienten abgestimmt, dass mit dem Schließen der Schnellverschlüsse 8 die Zugfederelemente 11a bis 11c unter die gewünschte Zugspannung gesetzt werden. Dadurch kann die dargestellte Osteoporose-Orthese in dynamischer Weise Torsions- oder Beugebewegungen des Oberkörpers des Patienten folgen und baut dabei jeweils entsprechende Gegenkräfte auf. Diese Gegenkräfte erhöhen sich mit dem Maß der entsprechenden Beuge- oder Torsionsbewegung des Oberkörpers, so dass der Oberkörper lediglich begrenzte Bewegungen durchführen kann. Die begrenzte Beweglichkeit ist auf die jeweilige Therapie des Patienten abgestimmt und gewährleistet, dass keine zu starken, für den Heilungs- oder Therapieprozess schädlichen Bewegungen durchgeführt werden können. Es ist in nicht dargestellter Weise möglich, die Längungsmöglichkeiten der Zugfederelemente 11a bis 11c über Anschläge zu begrenzen, so dass ab einer definierten Längung die Gurtabschnitte 4 bis 6 einschließlich der zugeordneten Zugfederelemente 11 a bis 11 c wie unelastische Gurtbänder wirken.

Anstelle von metallischen Zugfederelementen können auch andere Arten von Federelementen, wie insbesondere elastische Bänder oder Stränge aus Kunststoff- oder Textilmaterialien oder auch aus Gummi vorgesehen sein. Die Zugfederelemente 11a bis 11c können - analog zu den Schnellverschlüssen 8 - mit parallel angeordneten Verbindungsstreifen kombiniert sein, die flexibel, aber unelastisch ausgeführt sind und eine gestreckte Länge aufweisen, die der maximal zulässigen Streckung des entsprechenden Zugfederelementes entspricht. Sobald daher die maximale Streckung des jeweiligen Zugfederelementes überschritten wird, wird eine entsprechende Zugkraft der Gurtabschnitte über die jetzt straffen und unelastischen Verbindungsstreifen übertragen, so dass - wie beschrieben - die Gurtabschnitte als unelastische Rückhaltegurte wirken.

## Patentansprüche

1. Orthopädische Stützeinrichtung für einen menschlichen Rücken-und Lumbalbereich mit einer Lumbalbandage sowie mit einer Trägergurtanordnung, die wenigstens einen dorsalen Gurtabschnitt sowie zwei über Schulterbereiche geführte Gurtabschnitte umfasst, und die mit der Lumbalbandage verbunden ist, wobei wenigstens einem Gurtabschnitt (4, 5) ein elastisch nachgiebiger Kraftbegrenzungsteil (11a bis 11c) zugeordnet ist, **dadurch gekennzeichnet, dass** der Kraftbegrenzungsteil (11a bis 11c) im Bereich der Verbindung des Gurtabschnittes (4, 5) mit der Lumbalbandage (1) angeordnet ist.

2. Stützeinrichtung nach Anspruch 1, **dadurch gekennzeichnet, dass** der Kraftbegrenzungsteil ein Zug- oder Druckfederelement (11a bis 11c) umfasst.

3. Stützeinrichtung nach Anspruch 1, **dadurch gekennzeichnet, dass** dorsal mit der Lumbalbandage (1) eine zumindest weitgehend formsteife Stützplatte (3) verbunden ist, an der die dorsalen Gurtabschnitte (4, 5) angreifen.

4. Stützeinrichtung nach wenigstens einem der vorhergehenden Ansprüche, **dadurch gekennzeichnet, dass** der wenigstens eine Kraftbegrenzungsteil (11 a bis 11c) austauschbar zwischen der Stützplatte (3) und dem zugehörigen Gurtabschnitt (4, 5) angeordnet ist.

5. Stützeinrichtung nach wenigstens einem der vorhergehenden Ansprüche, **dadurch gekennzeichnet, dass** wenigstens der Gurtabschnitt (4, 6), dem der Kraftbegrenzungsteil (11a bis 11c) zugeordnet ist, unelastisch ausgeführt ist.

6. Stützeinrichtung nach wenigstens einem der vorhergehenden Ansprüche, **dadurch gekennzeichnet, dass** Mittel zur Einstellung einer Zugspannung des wenigstens einen Kraftbegrenzungsteiles (11a bis 11c) und des wenigstens einen zugeordneten Gurtabschnittes (4 bis 6) vorgesehen sind.

7. Stützeinrichtung nach Anspruch 6, **dadurch gekennzeichnet, dass** die Mittel zur Einstellung der Gurtspannung Mittel (13, 14) zur Längeneinstellung der Gurtabschnitte (4 bis 6) umfassen.

8. Stützeinrichtung nach Anspruch 6, **dadurch gekennzeichnet, dass** die Mittel zur Einstellung der Gurtspannung wenigstens einen Schnellverschluss (8) umfassen.

9. Stützeinrichtung nach Anspruch 8, **dadurch gekennzeichnet, dass** der Schnellverschluss (8) ein flexibler Verbindungsstreifen (9) zugeordnet ist, dessen Länge größer ist als eine Gesamtlänge des Schnellverschlusses (8), und der im Bereich des Schnellverschluss (8) benachbarte Endbereiche jeweils eines Gurtabschnittes (5, 6) miteinander derart verbindet, dass die Länge des Gurtabschnittes (5, 6) bei geöffnetem Schnellverschluss (8) größer ist als bei geschlossenem Schnellverschluss (8).

10. Stützeinrichtung nach Anspruch 8, **dadurch gekennzeichnet, dass** als Schnellverschluss (8) ein mechanischer Steck-/Rastverschluss vorgesehen ist, der in Längsrichtung des Gurtabschnittes (5, 6) werkzeuglos zu schließen oder zu öffnen ist.

## Claims

1. Orthopedic support device for a human back and lumbar region having a lumbar bandage and a carrying strap arrangement which comprises at least one dorsal strap section and two strap sections passing over shoulder regions and which is connected to the lumbar bandage, where at least one strap section (4, 5) is associated with an elastically yielding force limitation part (11a to 11c), **characterized in that** said force limitation part (11 a to 11c) is arranged in the area of connection of the strap section (4, 5) to the lumbar bandage (1).

2. Support device according to Claim 1, **characterized in that** the force limitation part comprises a tension or compression spring element (11 a to 11 c).

3. Support device according to Claim 1, **characterized in that** an at least largely form-rigid support pad (3) on which the dorsal strap sections (4, 5) act is dorsally connected to the lumbar bandage.

4. Support device according to at least one of the preceding claims, **characterized in that** the at least one force limitation part (11a to 11c) is arranged replaceably between the support pad (3) and the associated strap section (4, 5).

5. Support device according to at least one of the preceding claims, **characterized in that** at least the strap section (4, 6) with which the force limitation part (11 a to 11 c) is associated is designed non-elastic.

6. Support device according to at least one of the preceding claims, **characterized in that** means are provided for setting a tension of the at least one force limitation part (11a to 11c) and of the at least one associated strap section (4 to 6).

7. Support device according to Claim 6, **characterized in that** the means for setting the belt tension comprise means (13, 14) for setting the length of the strap sections (4 to 6).

8. Support device according to Claim 6, **characterized in that** the means for setting the belt tension comprise at least one quick-action fastener (8).

9. Support device according to Claim 8, **characterized in that** the quick-action fastener (8) is associated with a flexible connecting strip (9) whose length is greater than a total length of the quick-action fastener (8) and which in the area of the quick-action fastener (8) connects adjacent end areas of each strap section (5, 6) to one another such that the length of the strap section (5, 6) when the quick-action fastener (8) is opened is greater than when the quick-action fastener (8) is closed.

10. Support device according to Claim 8, **characterized in that** a mechanical insert/snap fastener is provided as the quick-action fastener (8) and can be closed or opened without tools in the longitudinal direction of the strap section (5, 6).

## Revendications

1. Dispositif de soutien orthopédique pour une zone dorsale et lombaire humaine, avec une ceinture lombaire ainsi qu'un dispositif de sangles qui comprend au moins une partie de sangle dorsale ainsi que deux parties de sangle passant au-dessus de la région des épaules, et qui est relié à la ceinture lombaire, sachant qu'un limiteur d'effort élastiquement flexible (11a à 11c) est affecté à au moins une partie de sangle (4, 5), **caractérisé en ce que** le limiteur d'effort (11a à 11c) est disposé dans la zone où la partie de sangle (4, 5) est reliée à la ceinture lombaire (1).

2. Dispositif de soutien selon la revendication 1, **caractérisé en ce que** le limiteur d'effort comprend un élément élastique de pression ou de traction (11a à 11c).

3. Dispositif de soutien selon la revendication 1, **caractérisé en ce qu'**une plaque de soutien (3) au moins essentiellement rigide est reliée dans le dos à la ceinture dorsale (1), plaque dans laquelle s'engagent les parties de sangle (4, 5) dorsales.

4. Procédé selon au moins l'une des revendications précédentes, **caractérisé en ce que** le limiteur d'effort (11a à 11c) au moins en présence est disposé de manière à pouvoir être remplacé entre la plaque de soutien (3) et la partie de sangle (4, 5) correspondante.

5. Procédé selon au moins l'une des revendications précédentes, **caractérisé en ce qu'**au moins la partie de sangle (4, 6) à laquelle est affecté le limiteur d'effort (11a à 11c) présente une conception non élastique.

6. Dispositif de soutien selon au moins l'une des revendications précédentes, **caractérisé en ce que** sont prévus des moyens pour régler une tension de l'au moins un limiteur d'effort (11a à 11e) et de l'au moins une partie de sangle correspondante (4 à 6).

7. Dispositif de soutien selon la revendication 6, **caractérisé en ce que** les moyens de réglage de la tension de la sangle comprennent des moyens (13, 14) de réglage de la longueur des parties de sangle (4 à 6).

8. Dispositif de soutien selon la revendication 6, **caractérisé en ce que** les moyens de réglage de la tension de la sangle comprennent au moins un système de fermeture rapide (8).

9. Dispositif de soutien selon la revendication 8, **caractérisé en ce qu'**est affectée au système de fermeture rapide (8) une bande de liaison (9) flexible dont la longueur est supérieure à une longueur totale du système à fermeture rapide (8), et qui, dans la zone du système de fermeture rapide (8), relie entre elles les extrémités voisines de chaque partie de sangle (5, 6) de telle manière que la longueur de la partie de sangle (5, 6) est plus importante lorsque le système de fermeture rapide (8) est ouvert que lorsque le système de fermeture rapide (8) est fermé.

10. Dispositif de soutien selon la revendication 8, **caractérisé en ce qu'**est prévue, en tant que système de fermeture rapide (8), une fermeture à crans/pattes mécanique qui peut être ouverte ou fermée sans outil dans le sens longitudinal de la partie de sangle (5, 6).
